# EUROPEAN PATENT APPLICATION

(11) **EP 4 548 891 A1**
(43) Date of publication of application: **07.05.2025**
(21) Application number: 24210787.8
(22) Date of filing: 05.11.2024
(51) Int. Cl.: A61F 13/62, A44B 18/00, B29C 65/08

(54) **METHOD FOR FORMING CROSS-DIRECTIONALLY EXTENDING PROTRUSIONS IN A SUBSTRATE**

(30) Priority: 06.11.2023 US 202363596389 P
(71) Applicant: The Procter & Gamble Company, Cincinnati, OH 45202 (US)
(72) Inventor: LENSER, Todd Douglas, Cincinnati, 45202 (US); MYERS, Randall Allen, Cincinnati, 45202 (US)
(74) Representative: P&G Patent Germany

(57) **Abstract**

Methods and apparatuses for forming protrusions in a substrate by passing the substrate between recesses and a source of vibration energy. The protrusions may extend from the substrate in a direction transverse to the machine direction. The recesses may be formed via a plurality of inserts, some having one or more cavities defined in their outer surface. Absorbent articles may comprise such a substrate. The absorbent articles may have a central longitudinal axis, a central lateral axis, a substrate produced in a machine direction, a plurality of protrusions integrally formed in the substrate. The machine direction may be generally parallel to the longitudinal axis or to the lateral axis and a portion of the plurality of protrusions may extend in a direction transverse to the longitudinal axis or to the lateral axis.

## Description

### FIELD

The present disclosure relates generally to methods and apparatuses for altering a substrate and more specifically to methods and apparatuses for forming cross-directionally extending protrusions in a substrate.

### BACKGROUND

The discussion of shortcomings and needs existing in the field prior to the present disclosure is in no way an admission that such shortcomings and needs were recognized by those skilled in the art prior to the present disclosure.

Commercial processes exist for the ultrasonic formation of unitary hooks oriented in a machine direction MD. In the field of absorbent articles, such hooks may provide sufficient attachment force for use in a secondary fastener. Use of such hooks as a primary fastener, however, has been limited, because the attaching force is typically lower than required for a quality diaper closure. For primary fasteners on a diaper, micro-replicated or thermal formed hooks are commonly utilized. Thermal formed hooks may have a T shaped cross section, with the branches of the T disposed in a CD alignment relative to the making process. Such hooks provide significantly greater attachment forces to nonwovens, providing a more robust closure of a diaper. A need, therefore, exists for an improved process and product for unitary formed fasteners.

### SUMMARY

Various embodiments solve the above-mentioned problems and provide methods and devices useful for producing substrates comprising altered areas having protrusions extending in a cross direction or in a direction transverse to the machine direction, which may be suitable for use in absorbent articles even as primary fasteners. Various embodiments also provide substrates comprising such altered areas and absorbent articles formed therefrom as well as methods of making such articles.

Various embodiments relate to a method of altering a portion of a substrate comprising providing a first device having an outer surface and providing a second device having a source of vibration energy. The method may also comprise forming a nip between the source of vibration energy and the outer surface and conveying the substrate through the nip in a machine direction. Finally, the method may comprise altering a portion of the substrate in the nip to create altered areas integrally formed in the substrate. According to various embodiments, at least some of the altered areas extend from the substrate in a direction transverse to the machine direction. According to various embodiments, the first device may comprise a plurality of inserts, each of the inserts having an outer surface, wherein a plurality of cavities are defined in the outer surface of at least some of the inserts;

Various embodiments relate to an absorbent article comprising a central longitudinal axis, a central lateral axis, a substrate produced in a machine direction, a plurality of protrusions integrally formed in the substrate. According to some embodiments, the machine direction may be generally parallel to the longitudinal axis and a portion of the plurality of protrusions may extend in a direction transverse to the longitudinal axis. According to other embodiments, the machine direction may be generally parallel to the lateral axis and a portion of the plurality of protrusions extend in a direction transverse to the lateral axis.

Various embodiments relate to a method of manufacturing a component of an absorbent article. The method may comprise providing a first device comprising an outer surface. providing a second device comprising a source of vibration energy, forming a nip between the source of vibration energy and the outer surface, conveying the substrate through the nip in a machine direction, and altering a portion of the substrate in the nip to create altered areas integrally formed in the substrate. According to some embodiments, a portion of the altered areas extend from the substrate in a direction transverse to the machine direction. The substrate may form the component of the absorbent article.

These and other features, aspects, and advantages of various embodiments will become better understood with reference to the following description, figures, and claims.

### BRIEF DESCRIPTION OF THE DRAWINGS

Many aspects of this disclosure can be better understood with reference to the following figures.
FIG. 1 is an example according to various embodiments illustrating a method of altering a portion of a substrate and an isometric view of an apparatus for altering a portion of a substrate.
FIG. 2 is an example according to various embodiments illustrating a partially exploded, isometric view of a portion of the first device.
FIG. 3 is an example according to various embodiments illustrating a partially exploded, isometric view of a portion of the first device.
FIG. 4 is an example according to various embodiments illustrating an isometric view of a portion of the first device.
FIG. 5 is an example according to various embodiments illustrating a side view of an insert having a continuous surface.
FIG. 6 is an example according to various embodiments illustrating a side view of an insert having a discontinuous surface.
FIG. 7A is an example according to various embodiments illustrating an isometric view of a stacked array of inserts comprising a plurality of inserts having discontinuous surfaces and a plurality of inserts having continuous surfaces.
FIG. 7B is an example according to various embodiments illustrating an isometric view of a ring from which inserts may be cut.
FIG. 8 is an example according to various embodiments illustrating a lower isometric view a first wedge anchor.
FIG. 9 is an example according to various embodiments illustrating a lower isometric a second wedge anchor.
FIG. 10 is an example according to various embodiments illustrating an isometric view of a base of a first device of the apparatus.
FIG. 11A is an example according to various embodiments illustrating a side view of the base of the first device.
FIG. 11B is an example according to various embodiments illustrating a cross-section along line 11-11 as shown in FIG. 11A.
FIG. 12A is an example according to various embodiments illustrating a front view of the first device of the apparatus.
FIG. 12B is an example according to various embodiments illustrating a cross-section along line 12-12 as shown in FIG. 12A.
FIG. 13A is an example according to various embodiments illustrating a side view of the first device of the apparatus.
FIG. 13B is an example according to various embodiments illustrating a cross-section along line 13-13 as shown in FIG. 13A.
FIG. 14 is an example according to various embodiments illustrating an isometric view of a substrate comprising a plurality of altered portions.
FIG. 15 is an example according to various embodiments illustrating an isometric view of a substrate comprising a plurality of altered portions.
FIG. 16 is an example according to various embodiments illustrating an isometric view of a substrate comprising a plurality of altered portions.
FIG. 17A is an example according to various embodiments illustrating a top view of a substrate comprising a plurality of altered portions.
FIG. 17B is an example according to various embodiments illustrating a cross-section along line 17-17 as shown in FIG. 17A.
FIG. 18 is an example according to various embodiments illustrating an isometric view of a portion of a substrate having a single protrusion.
FIG. 19A is an example according to various embodiments illustrating a top view of a portion of a substrate having a single protrusion.
FIG. 19B is an example according to various embodiments illustrating a cross-section along line 19-19 as shown in FIG. 19A.
FIG. 20 is an example according to various embodiments illustrating a top view of a portion of a substrate having a single protrusion.
FIG. 21 is an example according to various embodiments illustrating an absorbent article comprising a fastening member comprising protrusions extending in directions substantially transverse to the machine direction in which the substrate comprising the protrusions was formed.
FIG. 22 is an example according to various embodiments illustrating an absorbent article comprising one or more substrates.

It should be understood that the various embodiments are not limited to the examples illustrated in the figures.

### DETAILED DESCRIPTION

### Introduction and Definitions

This disclosure is written to describe the invention to a person having ordinary skill in the art, who will understand that this disclosure is not limited to the specific examples or embodiments described. The examples and embodiments are single instances of the invention which will make a much larger scope apparent to the person having ordinary skill in the art. Unless defined otherwise, all technical and scientific terms used herein have the same meaning as commonly understood by the person having ordinary skill in the art. It is also to be understood that the terminology used herein is for the purpose of describing examples and embodiments only, and is not intended to be limiting, since the scope of the present disclosure will be limited only by the appended claims.

All the features disclosed in this specification (including any accompanying claims, abstract, and drawings) may be replaced by alternative features serving the same, equivalent, or similar purpose, unless expressly stated otherwise. Thus, unless expressly stated otherwise, each feature disclosed is one example only of a generic series of equivalent or similar features. The examples and embodiments described herein are for illustrative purposes only and that various modifications or changes in light thereof will be suggested to the person having ordinary skill in the art and are to be included within the spirit and purview of this application. Many variations and modifications may be made to the embodiments of the disclosure without departing substantially from the spirit and principles of the disclosure. All such modifications and variations are intended to be included herein within the scope of this disclosure. For example, unless otherwise indicated, the present disclosure is not limited to particular materials, reagents, reaction materials, manufacturing processes, or the like, as such can vary. It is also to be understood that the terminology used herein is for purposes of describing particular embodiments only and is not intended to be limiting. It is also possible in the present disclosure that steps can be executed in different sequence where this is logically possible.

All numeric values are herein assumed to be modified by the term "about," whether or not explicitly indicated. The term "about" generally refers to a range of numbers that one of skill in the art would consider equivalent to the recited value (for example, having the same function or result). In many instances, the term "about" may include numbers that are rounded to the nearest significant figure.

In everyday usage, indefinite articles (like "a" or "an") precede countable nouns and noncountable nouns almost never take indefinite articles. It must be noted, therefore, that, as used in this specification and in the claims that follow, the singular forms "a," "an," and "the" include plural referents unless the context clearly dictates otherwise. Thus, for example, reference to "a support" includes a plurality of supports. Particularly when a single countable noun is listed as an element in a claim, this specification will generally use a phrase such as "a single." For example, "a single support."

Unless otherwise specified, all percentages indicating the amount of a component in a composition represent a percent by weight of the component based on the total weight of the composition.

Where a range of values is provided, it is understood that each intervening value, to the tenth of the unit of the lower limit (unless the context clearly dictates otherwise), between the upper and lower limit of that range, and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

In this specification and in the claims that follow, reference will be made to a number of terms that shall be defined to have the following meanings unless a contrary intention is apparent.

"Disposed on" refers to a positional state indicating that one object or material is arranged in a position adjacent to the position of another object or material. The term does not require or exclude the presence of intervening objects, materials, or layers.

"Absorbent article" refers to devices that absorb and contain liquid, and more specifically, refers to devices that are placed against or in proximity to the body of the wearer to absorb and to contain various exudates discharged from the body.

"Align" or "aligned" or "aligning" means to place or to arrange in a straight line. Aligning edges of substrates, therefore, means arranging the substrates so that the edges in question extend along approximately the same line. It is to be appreciated that aligning edges of substrates can be accomplished in a variety of ways, including placing the substrates one on top of the other or side by side.

"Facing relationship" refers to a relative positioning of materials, such as substrates, in which a surface of one material is oriented toward a surface of another material. For example, when two substrates are stacked on top of each other, they are in a facing relationship. The term does not require or exclude the presence of intervening objects, materials, or layers.

"Machine direction" (MD) refers to the direction of material flow through a process. In addition, relative placement and movement of material can be described as flowing in the machine direction through a process from upstream in the process to downstream in the process.

"Cross direction" or "cross machine direction" (CD) refers to a direction that is generally perpendicular to the machine direction.

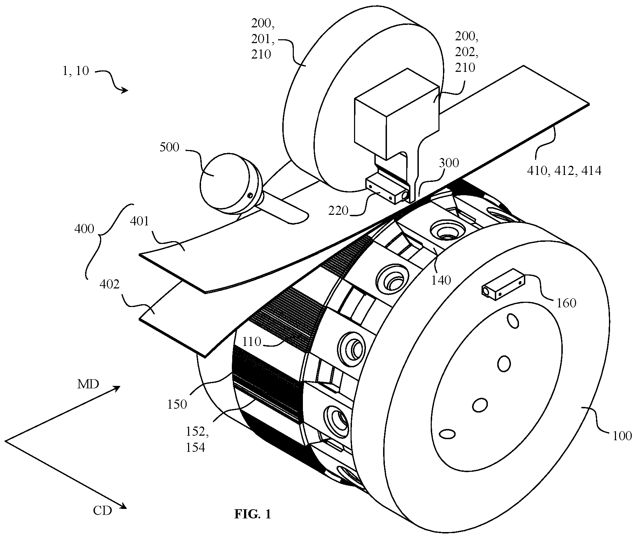

FIG. 1 is an example according to various embodiments illustrating a method 1 of altering a portion 410 of a substrate 400 and an isometric view of an apparatus 10 for altering a portion 410 of a substrate 400. The apparatus 10 may comprise at least a first device 100 and a second device 200. The first device 100 may comprise an outer surface 150. The second device 200 may comprise a source of vibration energy 210. The first device 100 and the second device 200 may be positioned to form a nip 300 between the outer surface 150 and the source of vibration energy 210. The second device 200 may comprise, for example, a rotary sonotrode 201 and/or a blade sonotrode 202. The source of vibration energy 210 may provide ultrasonic energy.

The substrate 400 may comprise one or more layers. For example, the substrate 400 may include a first layer 401 and a second layer 402. Each layer may comprise one or more materials. The layers may comprise the same or different materials. A layer may comprise a film, a nonwoven, a nonwoven with film layers, or other laminates. A layer may comprise a film with fibrous reinforcement, the reinforcement may comprise polymer fibers, a nonwoven, cellulose, or cellulosic fibers. A layer may comprise a nonwoven comprising at least one bi-component fiber. The bi-component fiber may comprise a first chemical species in a core region and a second chemical species in a sheath region. In alternate embodiments, the first and second chemical species may be disposed side by side, islands in the sea, eccentric, as sheets, or in other geometric relationships. The chemical species may comprise polyethylene (PE), polypropylene (PP), polyethylene terephthalate (PET), polyvinyl alcohol (PVA), or a variety of other polymers. The bi-component fiber may comprise a PE sheath adjacent a PP core. The bi-component fiber may comprise a PP sheath adjacent a PET core. The softening temp, glass transition temperature (Tg), and/or melting temperature of a first component may be different than the softening temp, Tg, and/or melting temperature of a second component. In some embodiments, a first component may comprise a film and second component may comprise a nonwoven. In some embodiments, a PE and/or PP film may be reinforced with PET fibers, which may have a melting, Tg, and/or softening temp higher than the PE or PP film.

An upstream heater 500 may optionally be employed to preheat a substrate 400 before the substrate 400 passes through the nip 300. For example, as shown in FIG. 1, upstream heater 500 may direct a heated fluid toward the outer portion of the substrate 400. The heated fluid may have a temperature in the range of about 90 °C to about 300 °C, or about 100 °C to about 275 °C, or about 100 °C to about 250 °C.

The first device 100 may optionally be cooled or heated via a cooling or heating apparatus 160. Similarly, the second device 200 may optionally be cooled or heated via a cooling or heating apparatus 220. Such cooling or heating apparatuses 160, 220 may be supplied with a fluid to transfer heat to or from the associated first or second device 100, 200. The fluid, according to any of these embodiments, may comprise water, air, glycol, a heat transfer oil, combinations thereof, or other suitable heat transfer fluids. The fluid type may be chosen to avoid cavitation. The fluid may be filtered to remove impurities which may cause cavitation. The fluid may be pressurized to prevent or minimize cavitation. The fluid may be chilled or heated. The fluid may be chilled or temperature controlled to -80°C to 20°C, -25°C to 20°C, -24°C to 5°C, or -16°C to +1°C, specifically reciting all 1°C increments within the specified ranges and all ranges formed therein or thereby. The fluid may be chilled or temperature controlled within 5°C, or 2°C, of the localized dew point. Similarly, the fluid may be heated or temperature controlled to 20°C to 40°C, 40°C to 60°C, up to 162°C, or up to a melting temperature of at least one of the constituent substrate chemical species.

Various embodiments relate to a method 1 comprising altering a portion 410 of a substrate 400. Such a method 1 may comprise providing one or more subcomponents of the apparatus 10. For example, the method 1 may comprise providing a first device 100 having an outer surface 150 and providing a second device 200 having a source of vibration energy 210. The method may also comprise forming a nip 300 between the source of vibration energy 210 and the outer surface 150. The step of forming the nip 300 may comprise making various positional adjustments to the first device 100 and/or subcomponents thereof. Additionally or alternatively, the step of forming the nip 300 may comprise making various positional adjustments to the second device 200 and/or subcomponents thereof.

In addition to the vibration energy applied from the source of vibration energy 210, the method 1 may optionally comprise imparting thermal energy to a portion of the substrate 400 upstream of the nip 300 to heat the portion of the substrate 400 to a temperature near or below a melting temperature of the portion of the substrate 400. The thermal energy may be applied in any suitable way. For example, a heater 500 may be employed to blow heated air at the substrate 400. The method 1 may optionally comprise cooling or heating the source of vibrational energy 210 and/or the first device 100. For example, a cooling or heating apparatus 220 may be disposed on or adjacent to the second device 200. Similarly, a cooling or heating apparatus 160 may be disposed on, in, or adjacent to the first device 100. Such a heating or cooling apparatus may comprise controlling the temperature of the first device 100 via cartridge heaters or via a heated and/or cooled fluid.

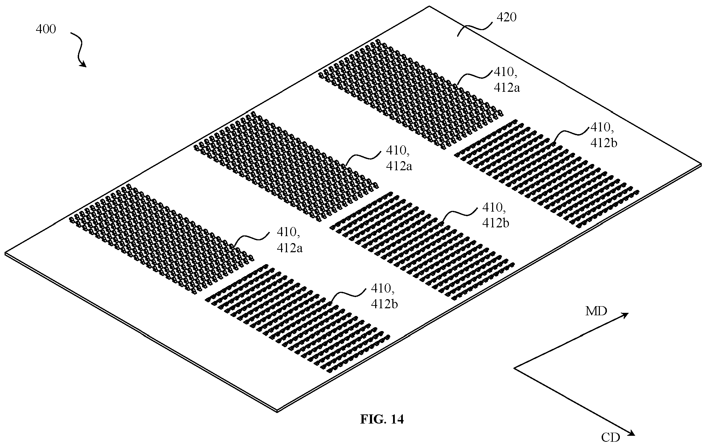

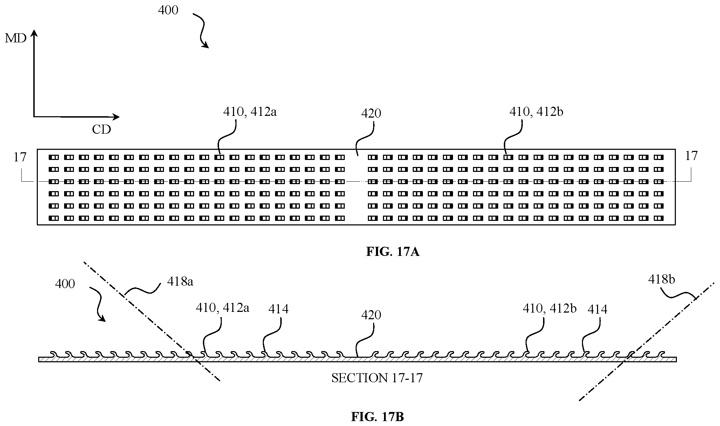

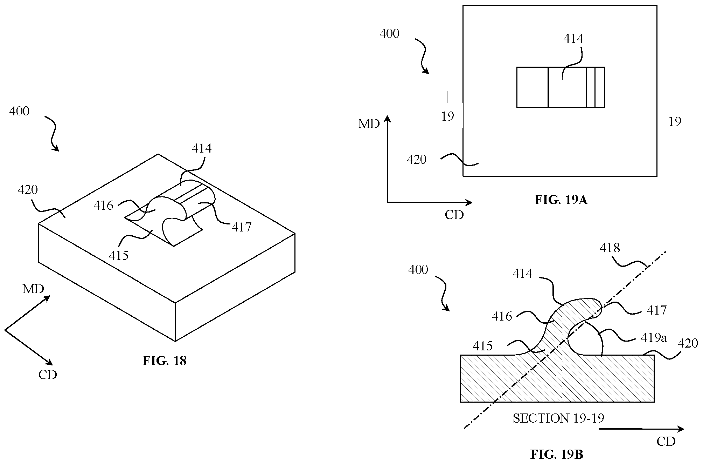

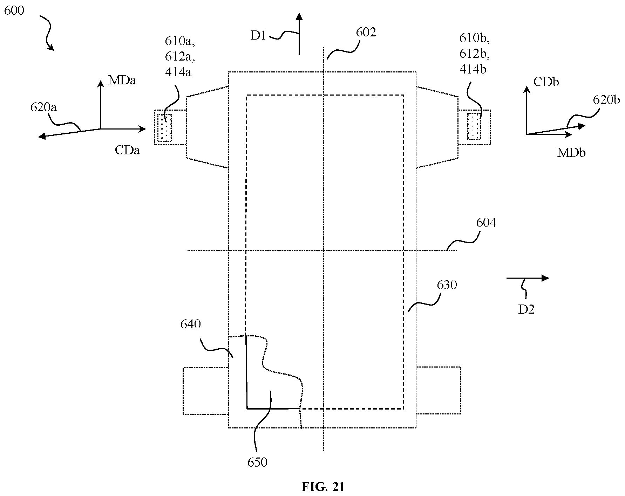

The method 1 may comprise conveying the substrate 400 through the nip 300 in a machine direction MD. Within the nip 300, the substrate 400 may be subjected to pressure and/or to vibration energy from the source of vibration energy 210. Therefore, the method 1 may comprise altering a portion 410 of the substrate 400 in the nip 300 to create altered areas 412 integrally formed in the substrate 400. In FIG. 1, the altered areas 412 are on the underside of the substrate and are not visible (See: FIGs. 14, 15, 16, 17A, and 17B). The altered areas 412 of the substrate 400 may have a variety of characteristics, which may be influenced by the outer surface 150 of the first device. For example, the outer surface 150 may comprise one or more cavities or recesses 152 into which a portion of the substrate 400 may be caused to flow upon the application of energy and/or pressure. The cavities or recesses 152 may have a shape 154 configured to produce protrusions 414 in the altered areas 412 of the altered portion 410 of the substrate 400. The structure and nature of the protrusions 414 will be discussed hereinafter. (See: FIGs. 14, 15, 16, 17A, and 17B for the general location of the protrusions 414 and FIGs. 18, 19A, 19B, and 20 for specific structural details of the protrusions 414). The method 1 may comprise providing the plurality of cavities or recesses 152 in the outer surface 150 of the first device 100, wherein the recesses 152 have a shape 154 configured to produce protrusions 414 in the altered areas 412 suitable for use in a fastener 610, such as a touch fastener useful in absorbent articles 600 (See: FIG. 21). Thus, in general, the method 1 may comprise forming protrusions 414 in a substrate 400 by passing the substrate between recesses 152 and a source of vibration energy 210. According to various embodiments, at least some of the altered areas 412 may comprise a protrusion 414 extending from the altered portion 410 of the substrate 400 in a direction 418 (See: FIGs. 17B, 19B, and 20) substantially transverse to the machine direction MD or substantially aligned with the cross direction CD.

Various embodiments comprise providing a stacked array 110 of inserts to form at least a portion of the outer surface 150 of the first device 100. The plurality of cavities or recesses 152 having the shape 154 configured to produce the protrusions 414 may also be formed by the stacked array 110 of inserts. The stacked array 110 of inserts may be held in place by an anchoring system 140.

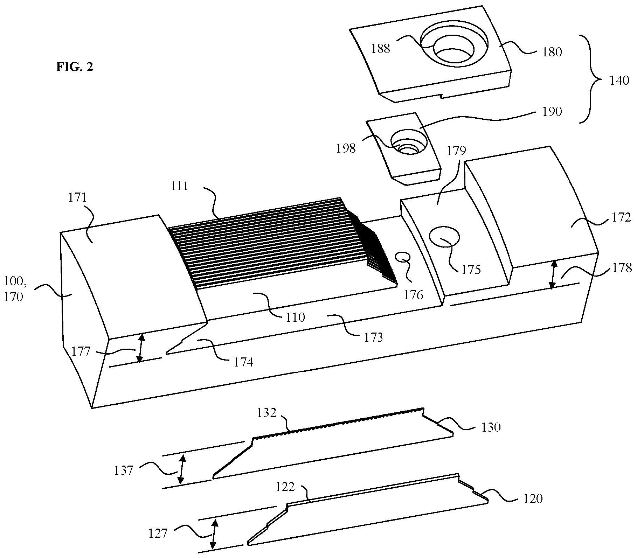

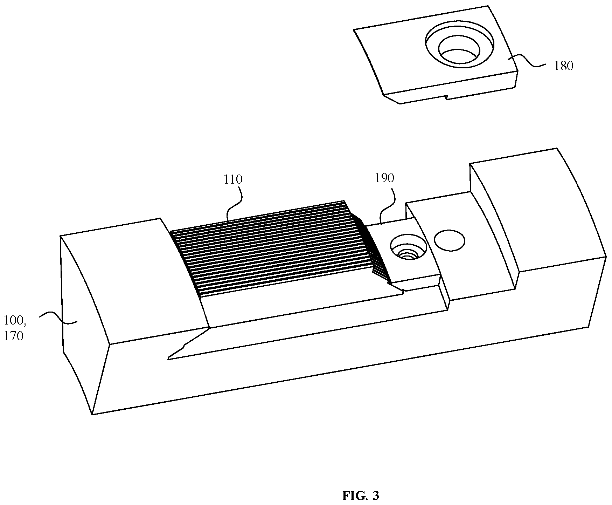

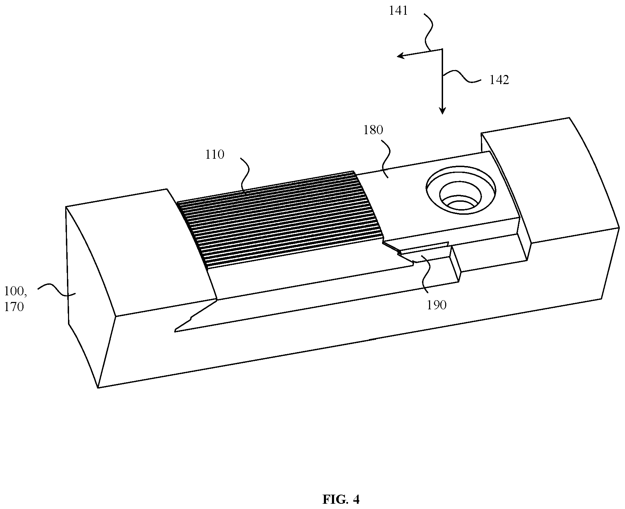

FIG. 2, FIG. 3, and FIG. 4 cooperate to a portion of the first device showing the stacked array 110 and the anchoring system 140. FIG. 2 provides a partially exploded, isometric view of the array 110 and the anchoring system 140 in association with a portion of the first device 100. The array 110 may comprise a plurality of inserts, including a first insert 120 and a second insert 130. More details about the inserts 120, 130 will be discussed hereinafter. Generally, the first insert 120 may have a continuous top surface 122 and the second insert 130 may have a discontinuous top surface 132. The plurality of inserts 120, 130 may be sandwiched together to form the stacked array 110, which may be disposed on a base 170 of the first device 100. The stacked array 110 may comprise an outer surface 111 that may be formed of or that may otherwise comprise the continuous top surface 122 of the first insert 120 and the discontinuous top surface 132 of the second insert 130. The array 110 may rest upon an interior platform surface 173 that is disposed between a first outer rim surface 171 and a second outer rim surface 172. The interior platform surface 173 may be offset relative to the first outer rim surface 171 by an first offset distance 177. Similarly, the interior platform surface 173 may be offset relative to the second outer rim surface 172 by a second offset distance 178. The first offset distance 177 by be the same as, substantially the same as, or different than the second offset distance 178. The first offset distance 177 and/or the second offset distance 178 may be the same as, substantially the same as, or different than a height 127 of the first insert 120 and/or a height 137 of the second insert 130. Thus, according to various embodiments the outer surface 111 of the stacked array may be substantially aligned with or coplanar with the first outer rim surface 171 and/or with the second outer rim surface 172. According to various embodiments the outer surface 111 of the stacked array may be offset from, such as by being elevated or depressed relative to, the first outer rim surface 171 and/or with the second outer rim surface 172. The offset may be about 0.5 mm, for example. The base may also comprise a groove 174 sized and shaped to accept the stacked array 110. The groove 174 may be angled from the first outer rim surface 171 to the interior platform surface 173 such that the stacked array 110 may be wedged into the groove 174 as shown. The opposite side of the stacked array 110 may be secured to the base 170 by an anchoring system 140 comprising a first wedge anchor 180 and a second wedge anchor 190. The second wedge anchor 190 may comprise a bolt hole 198 that may be aligned with a corresponding bolt hole 176 on the interior platform surface 173. Similarly, the first wedge anchor 180 may comprise a bolt hole 188 that may be aligned with a corresponding bolt hole 175 on the interior platform surface 173 or on an offset platform surface that is offset from the interior platform surface 173. It is to be appreciated that bolts or screws may be employed to secure the first wedge anchor 180 and the second wedge anchor 190 to the base 170. The second wedge anchor 190 may align an edge of cavity inset 132 against a first datum surface 173 and a second datum surface 174. A first wedge anchor 180 may compress wedge inserts 120 intermediate cavity inserts 130, thus forming a compressed monolithic stacked disk array 111. Compressive forces generated by wedge inserts 120 may prevent delamination of the disks in process. A final OD grind of the roll may align the outer diameter of inserts 120 and wedges 130 after the roll is assembled. It is also to be appreciated that any other suitable means of securing the wedge anchors 180, 190 to the base 170 may be employed. FIG. 3 shows a configuration in which the stacked array 110 is initially secured to the base 170 by the second wedge anchor 190. FIG. 4 shows a configuration in which the stacked array 110 is secured to the base 170 by both the second wedge anchor 190 and the first wedge anchor 180. Each wedge anchor 180, 190 provides a clamping force having components in a first anchoring direction 141 and a second anchoring direction 142.

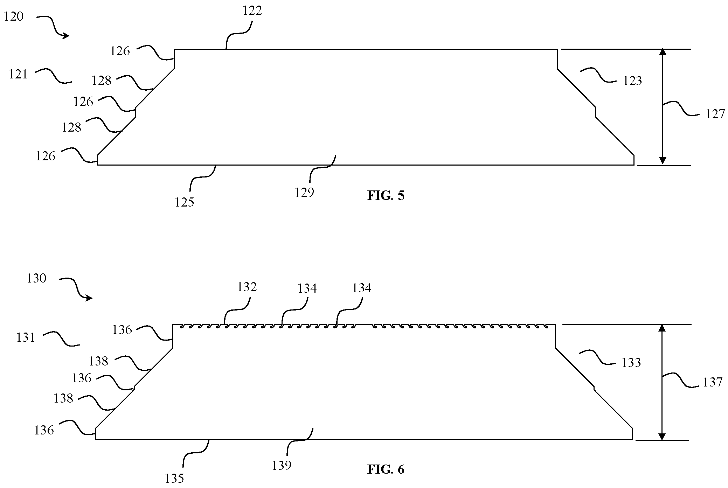

FIG. 5, FIG. 6, and FIG. 7A cooperate to illustrate details of the stacked array 110. FIG. 5 is an example according to various embodiments illustrating a side view of a first insert 120 having a continuous top surface 122. The first insert 120 may also have a base surface 125 opposing the continuous top surface 122 that is parallel or substantially parallel thereto and that is disposed a height 127 therefrom. The base surface 125 may optionally be curved to correspond with the curvature of the interior platform surface 173 of the base 170 of the first device 100. The continuous top surface 122 may also be curved such that it and the base surface 125 constitute a pair of curved, parallel planes. The first insert 120 may also have a first side 121 and a second side 123 opposing the first side. The first side 121 and the second side 133 may comprise one or more vertical engagement surfaces 126 and/or one or more angled engagement surfaces 128. The vertical and angle engagement surfaces 126, 128 may cooperate to engage the groove 174 of the base 170 and/or to engage the anchoring system 140. Finally, the first insert 120 may have a pair of opposing faces 129, which may be sandwiched against corresponding faces of other inserts. It is to be appreciated that the other inserts may structurally the same as or structurally different than the first insert 120 or the second insert 130. Any combination of inserts may be employed to provide the desired pattern of altered areas 412 and unaltered areas 420 in a substrate 400.

The inserts 120, 130 may be plates cut from precision shim stock, via methods common in the industry such as laser cutting, water jet cutting, milling, plunge EDM (electro discharge machining), electro-corrosive machining, wire EDM, turning, broaching, electron beam drilling, or grinding. For some patterns of hook elements, spacer inserts 120 having a continuous top surface 122 may be placed intermediate the recess inserts 130 having a discontinuous top surface 132. Such spacer insert 120 may be flat. According to some embodiments, the inserts 120, 130 are not flat, but instead are cut from a disk. The cut through the disk may be radially disposed from the axis of revolution.

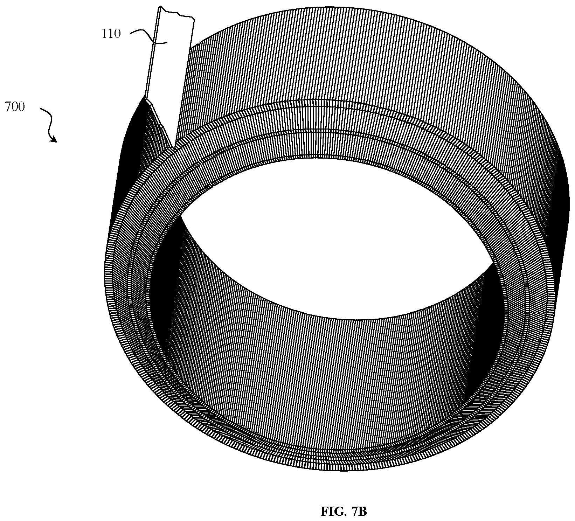

FIG. 7B is an example according to various embodiments illustrating an isometric view of a ring from which inserts may be cut. As shown in the FIG. 7B, such a cutout forms a rectangular cutout in the disk, resulting in a wedge shape at an end of the disk. The width of thed cuts preferably matches the thickness of the cavity plates. By cutting a plurality of wedges, assembling the spacer plates and the cavity plates, a segment of a mold roll may be formed. For patches of intermittent hook formation, thicker arcuate spacers may be cut. For example, a cavity plates may be 0.125mm thick, a spacer plate 0.5mm thick, and a spacer intermediate the patches may be 30mm circumferential length. Alternately, the cavity plates may be cut first from a ring or sector of a ring, with flat or wedge shaped spacers. The cut line need not be purely radial but may be angled or profiled to increase clamping pressure intermediate adjacent cavity plates, thin spacers plates, and patch spacers. The clamping pressure may seal between cavity plates and spacers during the molding process. The cuts in the spacer are shown in Fig. 7B as through cuts, creating a plurality of spacer wedge-shaped plates. Alternately, such cuts may only extend from one side or in a central region of the disk, with cavity plates filling the cutouts created. The precursor plate for spacers may be machined prior to cutting spacers. Such machining may include turning to form an inner periphery, and outer periphery, a lateral side, and/or one of more lateral angled surfaces.

Such angled surfaces may operatively engage with a gib, dovetail, or clamping element of the assemblage. The tool holder comprises an inner surface, and outer surface, a lateral edge, and a plurality of arctuate wedge shaped clamps. The clamps are tightened affixed to the tool holder by machine screws. In other embodiments, the tool holder may be machined directly into the outer surface of a mold roll mounting shaft. Combining multiple machine elements into a single part may reduce the tolerance stackup and machining complexity. In other embodiments, the inner surface of the tool holder may be tapered to ensure concentric mounting on a complementary tapered surface of a mold roll shaft. In such case, and radial tolerance variation becomes a small CD variation but the outer diameter remains consistent.

The clamping elements may mechanically register the cavity shims inner surface against the outer diameter of the tool holder or shaft with integrated tool holder. Cavity shims, inter-hook spacers, and inter-pattern spacers may be clamped concurrently in one method. In another method, all elements may be loosely mounted around the holder, partially tightened, a first plurality of clamps for the cavity shims tightened, then a second plurality of clamps for the spacers tightened. The first tightening may register, or mechanically align, the cavity shims against the holder OD. The second tightening may register the spacer disks against the tool holder OD, against a lateral edge, and/or against gib, wedge, or dovetail element. The spacer rings may have an ID slightly greater than the ID of the cavity shims. The cavity shims may thus be ensured to contact the OD of the central holder. The wedge may thus be ensured to provide a circumferential clamping force. The inter-hook spacers or other element may have a provision for a keyway, alignment pin, or registration edge which provides rotational locking to the rotary shaft or tool holder. The inter-hook spacer wedges may have an OD matching the OD of the cavity shims. The inter-patch spacers may have a smaller OD than the cavity shims in at least one region and regions at one or more ends substantially matching or slightly less than the OD of the assembled cavity shims. Any of the cavity shims, the spacer wedges, and/or the inter-hook spacers may have angled regions for operative contact with the clamping elements. The angled surfaces may form a gib or dovetail, forcing the cavity shims against the holder OD. The cavity shims may have a longer width in a cross machine direction than the spacer elements and may thus extend slightly past the wedges in at least one region. A first clamp may thus operate on the cavity spacers without affecting the spacer elements. In a second region, the cavity shims may be flush or recessed from surface of the spacer wedges. A second clamping means may operatively engage the spacers in this second region. To ensure cylindricity and concentricity, the various elements disclosed here may be mounted on a master roll and surface ground to a precision OD or ID.

In an alternate embodiment, a taper or wedge shape is formed in a disk, which may be annular. Such machining may be via turning and/or surface grinding. The disk is then cut into a plurality of segments. Such segments may be utilized as spacer elements or cavity plates. In the latter case, hooks recesses may be cut in a periphery prior or after cutting into segments. Such formation may be preferential for an alternate MD embodiment of the dovetail mounting method disclosed.

In an alternate embodiment the plurality of cavity plates and spacer plates is provided with one or more through cutouts. Machine screws, bolts or equivalent through these cutouts may clamp the plurality of plates together. End plates may be provided at one or multiple edges of the stack of plates. The end plates may be flat or have a tapered element. The end plates may be protrusions from a roll or tool holder. Such end plates may have a small clearance from the OD datum of the tool holder or shaft. Clamping to the rotary holder may be via clamping wedges or radial screw elements. In some embodiments a through hole may be provided for alignment pins or equivalent fixturing elements. Such fixturing may occur in a central region of the shims or at an edge. The clamping forces to align the cavity plates to the tool holder may be via these alignment elements.

In some embodiments, a nominally CD mounting arrangement may be utilized with the cavity plates mounted at an angle. The cutouts in the precursor ring for the wedges may be made at an angle. The inner surface of the cavity shims may be cut with a curved surface to match the OD of the tool holder element. The hooks formed by such tooling may be disposed at an angle intermediate a pure CD or MD alignment.

FIG. 6 is an example according to various embodiments illustrating a side view of a second insert 130 having a discontinuous top surface 132. The discontinuous top surface 132 may be discontinuous in that it comprises a plurality of cavities 134 arranged thereover. The plurality of cavities 134 may be shaped to produce the desired protrusions 414. The second insert 130 may also have a base surface 135 opposing the discontinuous top surface 132 that is parallel or substantially parallel thereto and that is disposed a height 137 therefrom. The base surface 135 may optionally be curved to correspond with the curvature of the interior platform surface 173 of the base 170 of the first device 100. The discontinuous top surface 122 may also be curved such that it and the base surface 135 constitute a pair of curved, parallel planes. The second insert 130 may also have a first side 131 and a second side 133 opposing the first side. The first side 131 and the second side 133 may comprise one or more vertical engagement surfaces 136 and/or one or more angled engagement surfaces 138. The vertical and angle engagement surfaces 136, 138 may cooperate to engage the groove 174 of the base 170 and/or to engage the anchoring system 140. Finally, like the first insert 120, the second insert 130 may have a pair of opposing faces 139, which may be sandwiched against corresponding faces of other inserts. It is to be appreciated that the other inserts may structurally the same as or structurally different than the first insert 120 or the second insert 130. Any combination of inserts may be employed to provide the desired pattern of altered areas 412 and unaltered areas 420 in a substrate 400.

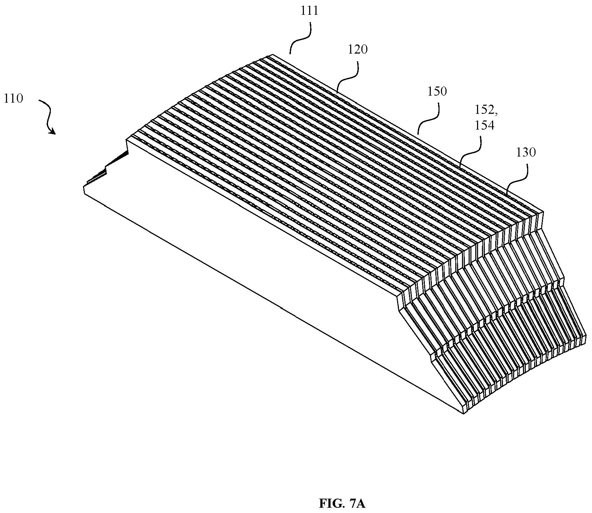

FIG. 7 is an example according to various embodiments illustrating an isometric view of a stacked array 110 comprising a plurality of inserts 130 having discontinuous surfaces 132 and a plurality of inserts 120 having continuous surfaces 122. The discontinuous surfaces 132 and the continuous surfaces 122 may cooperate to form an outer surface 111 of the stacked array 110, which may form all or a portion of the outer surface 150 of the first device 100. When sandwiched together, the first inserts 120 and the second inserts 130 form a plurality of recesses 152 having a shape 154 that may be configured to produce protrusions in an altered area 412 of a substrate 400.

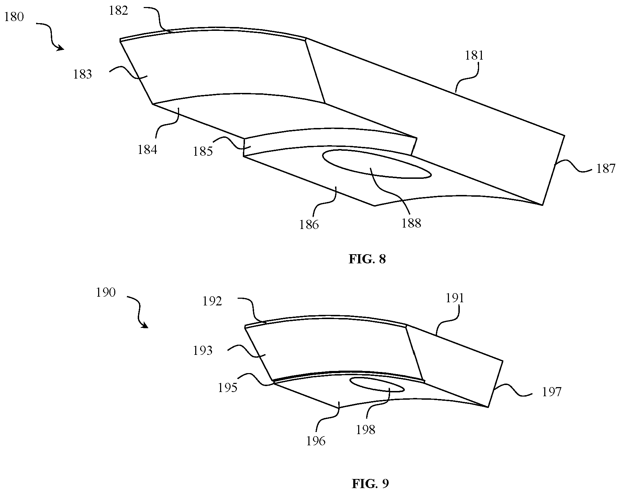

FIG. 8 and FIG. 9 cooperate to illustrate details of the anchoring system 140. FIG. 8 is an example according to various embodiments illustrating a lower isometric view a first wedge anchor 180. The first wedge anchor 180 may comprise a top portion 181. The top portion 181 may have a curvature corresponding to the curvature of the stacked array 110 and/or to the curvature of the base 170 of the first device 100. The top portion 181 may extend between a front edge 182 and an opposing rear surface 187. A base-abutment surface 186 may oppose the top portion 181. A bolt hole 188 may extend through the first wedge anchor 180 from the top portion 181 to the base-abutment surface 186. The base-abutment surface 186 may have a curvature corresponding to and/or parallel to the curvature of the top portion 181. A series of surfaces may be disposed between the front edge 182 and the base-abutment surface 186. For example, a first insert-abutment surface 183, a wedge-abutment surface 184, and a second insert-abutment surface 185 may be disposed between the front edge 182 and the base-abutment surface 186. The wedge-abutment surface 184 may have a curvature corresponding to and/or parallel to the curvature of the top portion 181. The wedge-abutment surface 184 may be configured to engage, to abut, or to otherwise press against a top portion 191 of the second wedge anchor 190 when both the first wedge anchor 180 and the second wedge anchor 190 are secured to the first device 100 as shown, for example, in FIG. 4, thereby applying a force in the first anchoring direction 141 and/or in the second anchoring direction 142.. The front edge 182, the first insert-abutment surface 183 and the second insert-abutment surface 185 may be configured to engage or to abut or to otherwise press against the plurality of vertical engagement surfaces 126, 136 and/or the plurality of angled engagement surfaces 128, 138 of the first or second insert 120, 130 or a stacked array 110 thereof, thereby applying a force in the first anchoring direction 141 and/or in the second anchoring direction 142.

FIG. 9 is an example according to various embodiments illustrating a lower isometric a second wedge anchor 190. The second wedge anchor 190 may comprise a top portion 191. The top portion 191 may have a curvature corresponding to the curvature of the stacked array 110 and/or to the curvature of the base 170 of the first device 100. The top portion 191 may extend between a front edge 192 and an opposing rear surface 197. A base-abutment surface 196 may oppose the top portion 191. A bolt hole 198 may extend through the second wedge anchor 190 from the top portion 191 to the base-abutment surface 196. The base-abutment surface 196 may have a curvature corresponding to and/or parallel to the curvature of the top portion 191. A series of surfaces may be disposed between the front edge 192 and the base-abutment surface 196. For example, a first insert-abutment surface 193 and a second insert-abutment surface 195 may be disposed between the front edge 192 and the base-abutment surface 196. The front edge 192, the first insert-abutment surface 193 and the second insert-abutment surface 195 may be configured to engage or to abut or to otherwise press against the plurality of vertical engagement surfaces 126, 136 and/or the plurality of angled engagement surfaces 128, 138 of the first or second insert 120, 130 or a stacked array 110 thereof, thereby applying a force in the first anchoring direction 141 and/or in the second anchoring direction 142.

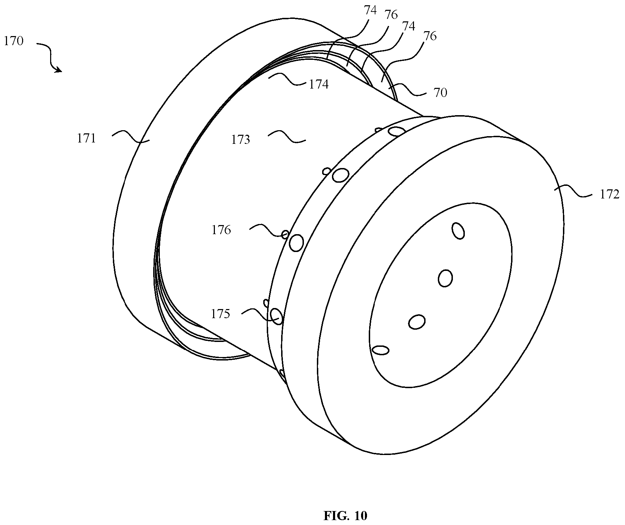

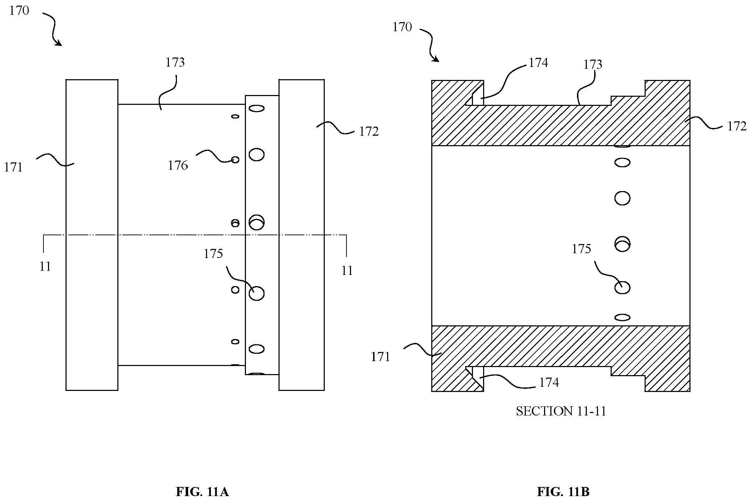

FIG. 10, FIG. 11A, and FIG. 11B cooperate to illustrate details of the base 170 of the first device 100. FIG. 10 is an example according to various embodiments illustrating an isometric view of the base 170 of the first device 100 of the apparatus 10. FIG. 11A is an example according to various embodiments illustrating a side view of the base 170 of the first device 100. FIG. 11B is an example according to various embodiments illustrating a cross-section along line 11-11 as shown in FIG. 11A. The groove 174 formed between the first outer rim surface 171 and the interior platform surface 173 may comprise an inner surface 70 having both vertical surfaces 74 and angled surfaces 76, which may correspond with and engage the vertical and angled engagement surfaces 126, 128, 136, 138 of the inserts 120, 130.

The tool holder and mold roll tooling may form an uneven outer surface. A cylindrical cover element may be used to provide a nominally cylindrical outer surface. The outer diameter of the cover may provide a uniform supporting surface for the substrate, especially where the substrate wraps the mod roll or has a tangent entry to the mold roll. The cylindrical cover may nominally smaller OD than the pattern elements or may be recessed slightly to avoid operative contact with the sonotrode. The outer cover element may have slightly increased diameter on an outboard or inboard edge, such as a step or taper to pull the web into a flat disposition and remove any wrinkles. The cylindrical cover may be split into multiple sections which form a substantially continuous outer surface once assembled. Keys, alignment pins or other elements may provide a locating function for one of more of the plurality of inserts. Such fixturing elements may provide a reaction force in a circumferential direction perpendicular to 141 and/or 142.

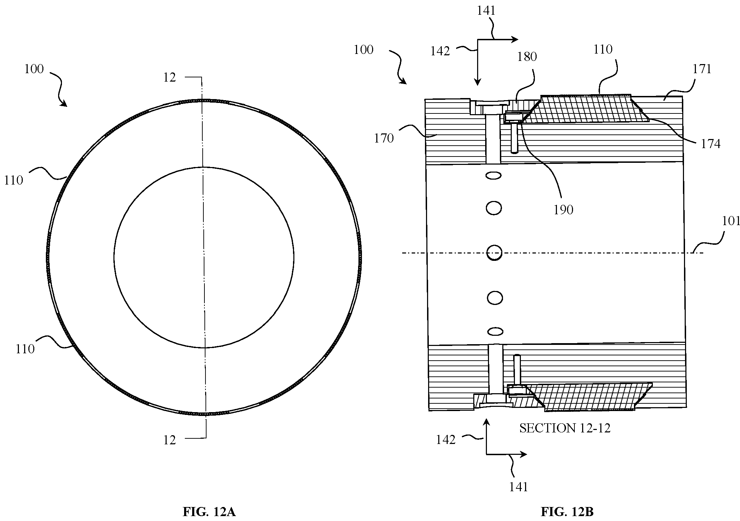

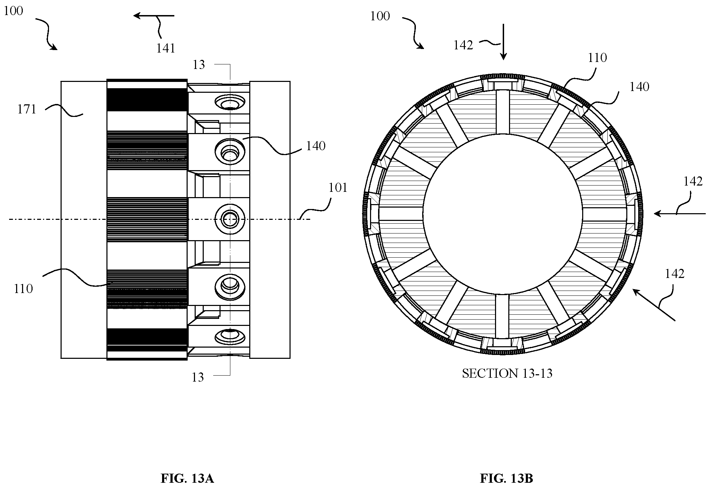

FIG. 12A, FIG. 12B, FIG. 13A, and FIG. 13B cooperate to illustrate details of how the base 170 of the first device 100, each stacked array 110 of inserts, and each anchoring system 140 may interact according to various embodiments. FIG. 12A is an example according to various embodiments illustrating a front view of the first device of the apparatus. FIG. 12B is an example according to various embodiments illustrating a cross-section along line 12-12 as shown in FIG. 12A. FIG. 13A is an example according to various embodiments illustrating a side view of the first device of the apparatus. FIG. 13B is an example according to various embodiments illustrating a cross-section along line 13-13 as shown in FIG. 13A. Each anchoring system may impart a first force in a first anchoring direction 141 and a second force in a second anchoring direction 142. The first anchoring direction 141 for each anchoring system 140 may be parallel to a central axis 101 of the first device 100 and may bias the stacked array 110 of inserts into the insert-accepting groove 174 of the base 170. The second anchoring direction 142 for each anchoring system 140 may be perpendicular to the central axis 101 and may bias the stacked array 110 of inserts toward the interior platform 173 of the base 170 of the first device 100.

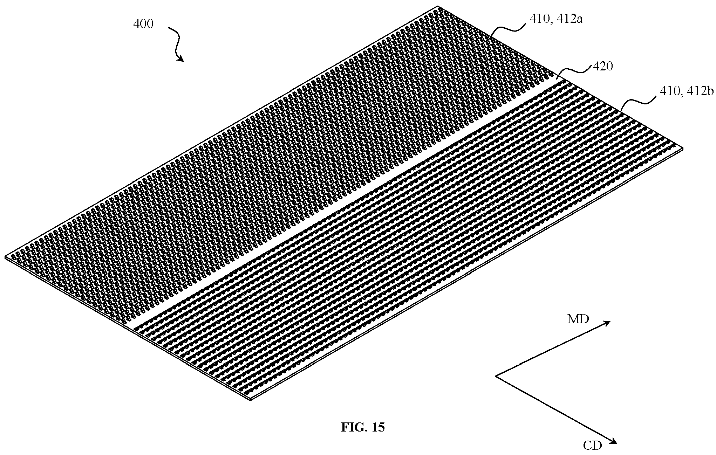

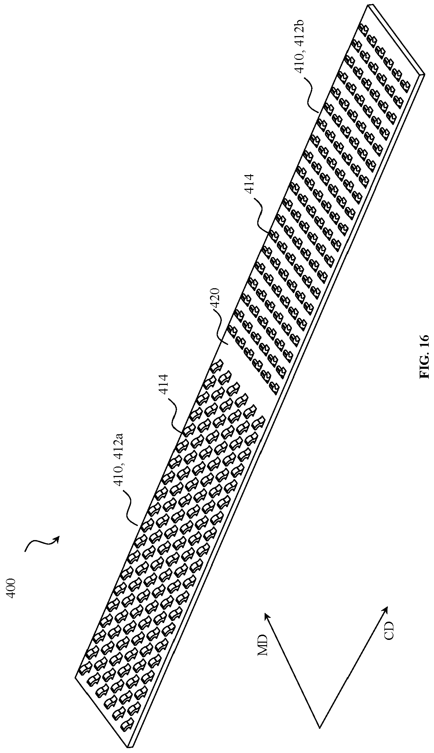

FIG. 14, FIG. 15, FIG. 16, FIG. 17A, and FIG. 17B cooperate to illustrate altered portions 410 and unaltered portions 420 of a substrate 400. The altered portions 410 included first altered areas 412a with protrusions 414 extending in a first extension direction 418a and second altered areas 412b with protrusions 414 extending in a second extension direction 418b. FIG. 14 is an example according to various embodiments illustrating an isometric view of a substrate 400 comprising a plurality of altered portions 410. As shown in FIG. 14 the altered portions 410 may be formed periodically on the substrate 400 such that they are separated by unaltered surface portions 420. Any pattern or arrangement may be employed. As shown in FIG. 15, the altered portions 410 may also be applied continuously across the substrate 400 in a machine direction MD. Multiple continuous altered portions 410 may also be continuous in the cross direction CD or may be separated by one or more unaltered surface portions 420. FIG. 16 shows an isometric view showing the protrusions 414 extending in different directions that are transverse to the machine direction MD. The first and second extension directions 418a, 418b are best seen in FIG. 17B, which shows a cross-section along line 17-17 as shown in FIG. 17A. Thus, according to various embodiments, at least some of the altered areas 412 may include protrusions 414 that extend from the substrate 400 in a direction 418 transverse to the machine direction MD. At least some of the altered areas 412 may include protrusions 414 that extend from the substrate 400 in a direction 418 generally perpendicular to the machine direction MD. Various embodiments may comprise conveying the substrate 400 through the nip 300 in a machine direction MD, wherein at least some of the protrusions 414 extend from the substrate 400 in a direction 418 transverse to the machine direction MD.

The protrusions 414 may have J or T cross section and may form hooks. FIG. 19B shows a J-shaped hook, for example. The protrusions 414 may be utilized as a primary fastener for an absorbent article 600, such as a diaper. The primary fastener may be formed in a nonwoven or film substrate. The primary fastener may be further assembled with a back ear of a diaper.

FIG. 18, FIG. 19A, FIG. 19B, and FIG. 20 cooperate to illustrate the structure and orientation of a protrusion 414 formed in an altered area 412 of an altered portion 410 of a substrate 400. FIG. 18 is an example according to various embodiments illustrating an isometric view of a portion of a substrate 400 having a single protrusion 414. The protrusion 414 may be surrounded by an unaltered surface portion 420 of the substrate 400. The protrusion 414 may have a base 415 disposed on or adjacent to the unaltered surface portion 420. The protrusion 414 may extend away from the surface 420 along a shaft 416 to a tip 417 in an extension direction 418.

FIG. 19A is an example according to various embodiments illustrating a top view of a portion of a substrate 400 having a single protrusion 414. FIG. 19B is an example according to various embodiments illustrating a cross-section along line 19-19 as shown in FIG. 19A. As shown in FIG. 19B, the extension direction 418 may be at an angle 419a relative to the unaltered surface 420 of the substrate 400. The angle 419a between the extension direction 418 of the protrusion 414 and the unaltered surface 420 of the substrate 400 may be from about 0 degrees to about 90 degrees, or about 5 degrees to about 85 degrees, or about 10 degrees to about 80 degrees, or about 15 degrees to about 75 degrees, or about 20 degrees to about 70 degrees, or about 25 degrees to about 65 degrees, or about 30 degrees to about 60 degrees, or about 35 degrees to about 55 degrees, or about 40 degrees to about 50 degrees, or about 45 degrees.

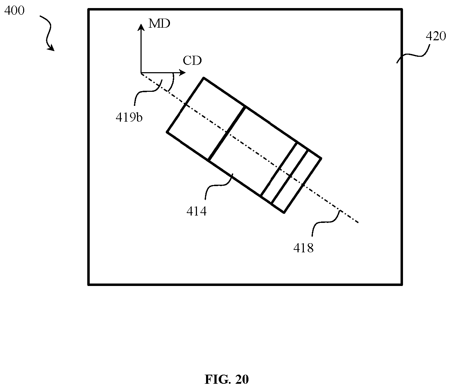

FIG. 20 is an example according to various embodiments illustrating a top view of a portion of a substrate 400 having a single protrusion 414. As shown in FIG. 20, the extension direction 418 may also be at an angle 419b relative to the cross machine direction CD in which the substrate 400 is formed. The angle 419b between the extension direction 418 of the protrusion 414 and the cross machine direction CD may be about 0 degrees to about 180 degrees, or about 5 degrees to about 175 degrees, or about 10 degrees to about 170 degrees, or about 15 degrees to about 165 degrees, or about 20 degrees to about 160 degrees, or about 25 degrees to about 155 degrees, or about 30 degrees to about 150 degrees, or about 35 degrees to about 145 degrees, or about 40 degrees to about 140 degrees, or about 45 degrees to about 135 degrees, or about 50 degrees to about 130 degrees, or about 55 degrees to about 125 degrees, or about 60 degrees to about 120 degrees, or about 65 degrees to about 115 degrees, or about 70 degrees to about 110 degrees, or about 75 degrees to about 105 degrees, or about 80 degrees to about 100 degrees, or about 85 degrees to about 95 degrees, or about 90 degrees.

FIG. 21 is an example according to various embodiments illustrating an absorbent article 600 comprising a first fastening member 610a and a second fastening member 610b. The first fastening member 610a comprises an altered portion 612a having protrusions 414a extending in directions 620a substantially transverse to a central longitudinal axis 602 of the absorbent article 600. The central longitudinal axis 602 is substantially or generally parallel to a machine direction MDa in which the first fastening member 610a was formed. The second fastening member 610b comprises a second fastening member 610b comprising an altered portion 612b having protrusions 414b extending in directions 620b substantially transverse to a central longitudinal axis 602 of the absorbent article 600. The central lateral axis 604 is substantially or generally parallel to a machine direction MDb in which the first fastening member 610a was formed. According to various embodiments, a majority of the plurality of protrusions 414a, 414b may extend in a direction transverse to the longitudinal axis 602. According to other embodiments, a majority of the plurality of protrusions 414a, 414b may extend in a direction transverse to the lateral axis 604.

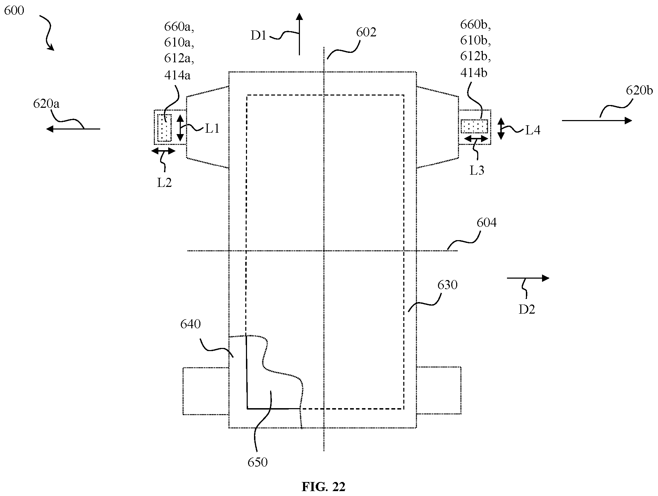

FIG. 22 is an example according to various embodiments illustrating an absorbent article 600 comprising one or more substrates 660a, 660b. The absorbent article 600 may comprise a liquid permeable topsheet 630, a liquid impermeable backsheet 640, and an absorbent core 650 positioned at least partially intermediate the topsheet 630 and the backsheet 640. The absorbent article 600 may also comprise a central longitudinal axis 602 extending in a first direction D1 and a central lateral axis 604 extending in a second direction D2. The second direction D2 may be perpendicular to the first direction D1. The absorbent article 600 may comprise a substrate 660a having a longer dimension L1 in the first direction D1 and a shorter dimension L2 in the second direction D2. The substrate 660a may comprise a plurality of integrally formed projections 414a extending in a direction 620a transverse to the first direction D1. The same absorbent article 600 or a different absorbent article 600 may comprise a substrate 660b having a longer dimension L3 in the second direction D2 and a shorter dimension L4 in the first direction D1. The substrate 660b may comprise a plurality of integrally formed projections 441b extending in a direction 620b transverse to the first direction D 1. The substate(s) 660a, 660b may comprise a fastening member 610a, 610, such as a fastener patch. The substrate(s) 660a, 660b may comprise a first elastic modulus in the first direction D1. The substrate(s) 660a, 660b may comprise a second elastic modulus in the second direction D2. The first elastic modulus may be greater than, less than, or equal to the second elastic modulus.

Various embodiments relate to a method 1 of manufacturing a component of an absorbent article 600. The component may be a fastening member such as the first fastening member 610a or the second fastening member 610b. As shown in FIG. 1, and as previously discussed, the method 1 may comprise providing a first device 100 comprising an outer surface 150, providing a second device 200 comprising a source of vibration energy 210, forming a nip 300 between the source of vibration energy 210 and the outer surface 150, conveying the substrate through the nip 300 in a machine direction MD, and altering a portion 410 of the substrate 400 in the nip to create altered areas 412 integrally formed in the substrate 400. According to various embodiments, a portion, such as protrusions 414, of the altered areas 412 extend from the substrate 400 in a direction 418 transverse to the machine direction MD. The substrate 400 with the altered areas 412 integrally formed may form the component of the absorbent article 600.

The methods and apparatus above may enable an improved product form. Unitary hooks formed from a NW are typically formed with an MD orientation. For low attachment strength applications, such designs may be sufficient. In other embodiments, such as the primary fastener for a diaper, it may be preferable to have some, all, or a majority of hooks with hooks facing in a preferred direction to resist loading. For a diaper primary fastener, hook patches may be preferentially formed in a CD orientation. Forming CD hooks rather than MD hooks may enable choice of a more advantageous hook shape. For example, in an MD hook may utilize a tulip shape (or other even shape) or an even mix of forward and backward oriented J hooks. By utilizing CD hooks, the hook field may be biased to have most or all of the hooks facing in a direction to oppose the primary loading forces. Additionally, the hook orientation may be optimized for regions. For example, the medial portion of a primary fastener may face in a direction to oppose contraction of an elastic element, which may comprise back ear, waist band, or leg cuff. The distal portion of the fastener hook field may face in an opposite direction. Such hooks may better oppose initial peel as the fastener is detached. These regions may include a mix of fastener orientations. In some embodiments, it may be advantageous to dispose the hooks at an inclined angle intermediate a CD or MD orientation. For example, a nominally CD hook orientation may be rotated several degrees such that the hooks better oppose the combined loading a waist contraction and a leg cuff contraction. In some embodiments a first region may have hooks aligned in a first orientation and a second region with hooks rotated in a second direction. A region may align with waist or back ear closure forces encircling a wearer's waist, which may comprise strand elastics, elastic film, extensible non-wovens, a front waist band, a rear waistband, a back ear, a front ear, an extensible side panel, or other extensible elements. A region may align with forces from a leg cuff elastomer, such as a strand or stretch film.

### Examples/Combinations

1. A method of forming protrusions in a portion of a substrate comprising:
   providing a first device, wherein the first device comprises a plurality of inserts, each of the inserts having an outer surface, wherein a plurality of cavities are defined in the outer surface of at least some of the inserts;
   providing a second device comprising a source of vibration energy;
   forming a nip between the source of vibration energy and the outer surfaces of the inserts;
   conveying the substrate through the nip; and
   forming protrusions in the substrate in the nip using the cavities and the source of vibration energy.
2. The method of Paragraph 1, comprising conveying the substrate through the nip in a machine direction, wherein at least some of the protrusions extend from the substrate in a direction transverse to the machine direction.
3. The method of Paragraph 1 or 2, comprising cooling or heating the source of vibrational energy or the first device.
4. The method of any one of Paragraphs 1-3, wherein the second device is a rotary sonotrode, and wherein the vibration energy is ultrasonic energy.
5. The method of any one of Paragraphs 1-4, wherein the substrate comprises more than one layer or more than one material.
6. The method of any one of Paragraphs 1-5, comprising imparting thermal energy to the portion of the substrate upstream of the nip to heat the portion of the substrate to a temperature below a melting temperature of the portion of the substrate.
7. The method of any one of Paragraphs 1-6, comprising anchoring the plurality of inserts to the first device in a first direction and in a second direction.
8. The method of any one of Paragraphs 1-7, comprising anchoring the plurality of inserts to the first device using a wedge.
9. An absorbent article comprising:
   a liquid permeable topsheet;
   a liquid impermeable backsheet;
   an absorbent core positioned at least partially intermediate the topsheet and the backsheet;
   a central longitudinal axis extending in a first direction;
   a central lateral axis extending in a second direction, wherein the second direction is perpendicular to the first direction;
   a substrate having a longer dimension in the first direction and a shorter dimension in the second direction; and
   the substrate comprising a plurality of integrally formed projections extending in a direction transverse to the first direction.
10. The absorbent article of Paragraph 9, wherein the substate comprises a fastener patch.
11. The absorbent article of Paragraph 9 or 10, wherein the substrate has a first elastic modulus in the first direction, wherein the substrate has a second elastic modulus in the second direction, and wherein the first elastic modulus is greater than the second elastic modulus.
12. An absorbent article comprising:
   a liquid permeable topsheet;
   a liquid impermeable backsheet;
   an absorbent core positioned at least partially intermediate the topsheet and the backsheet;
   a central longitudinal axis extending in a first direction;
   a central lateral axis extending in a second direction, perpendicular to the first direction;
   a substrate having a longer dimension in the second direction and a shorter dimension in the first direction; and
   the substrate comprising a plurality of integrally formed projections extending in a direction transverse to the first direction.
13. The absorbent article of Paragraph 12, wherein the substate comprises a fastener patch.
14. The absorbent article of Paragraph 12 or 13, wherein the substrate has a first elastic modulus in the first direction, wherein the substrate has a second elastic modulus in the second direction, and wherein the first elastic modulus is less than the second elastic modulus.
15. A method of manufacturing a component of an absorbent article comprising:
   providing a first device comprising an outer surface;
   providing a second device comprising a source of vibration energy;
   forming a nip between the source of vibration energy and the outer surface; conveying the substrate through the nip in a machine direction; and
   altering a portion of the substrate in the nip to create altered areas integrally formed in the substrate, wherein a portion of the altered areas extend from the substrate in a direction transverse to the machine direction;
   wherein the substrate with the altered areas integrally formed therein forms the component.
16. The method of Paragraph 15, wherein the at least some of the altered areas extend from the substrate in a direction generally perpendicular to the machine direction.
17. The method of Paragraph 15, wherein the at least some of the altered areas extend from the substrate in a direction in a range of about 45 degrees to about 135 degrees relative to the machine direction.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of altering a portion of a substrate comprising:
providing a first device comprising an outer surface;
providing a second device comprising a source of vibration energy;
forming a nip between the source of vibration energy and the outer surface; conveying the substrate through the nip in a machine direction; and
altering a portion of the substrate in the nip to create altered areas integrally formed in the substrate,
wherein at least some of the altered areas extend from the substrate in a direction transverse to the machine direction.

2. The method of Claim 1, wherein the at least some of the altered areas extend from the substrate in a direction generally perpendicular to the machine direction.

3. The method of Claim 1, wherein the at least some of the altered areas extend from the substrate in a direction in a range of about 45 degrees to about 135 degrees relative to the machine direction.

4. The method of any one of the preceding claims, comprising cooling or heating the source of vibrational energy or the first device.

5. The method of any one of the preceding claims, wherein the second device is a rotary sonotrode, and wherein the vibration energy is ultrasonic energy.

6. The method of any one of Claims 1 to 4, wherein the second device is a blade sonotrode, and wherein the vibration energy is ultrasonic energy.

7. The method of any one of the preceding claims, comprising providing a plurality of recesses in the outer surface of the first device, wherein the recesses have a shape configured to produce protrusions in the altered areas suitable for use in a fastener.

8. The method of any one of the preceding claims, wherein the substrate comprises more than one layer or more than one material.

9. The method of any one of the preceding claims, comprising imparting thermal energy to the portion of the substrate upstream of the nip to heat the portion of the substrate to a temperature near or below a melting temperature of the portion of the substrate.

10. The method of any one of the preceding claims, wherein the altered areas comprise protrusions suitable for use in a touch fastener.

11. The method of any one of Claims 1-6 or 8-10, wherein the first device comprises a plurality of inserts, each of the inserts having an outer surface, and wherein a plurality of cavities are defined in the outer surface of at least some of the inserts.
